# DEMANDE DE BREVET EUROPEEN

(11) **EP 4 804 205 A2**
(43) Date de publication de la demande: **09.09.2026**
(21) Numéro de dépôt: 26194144.7
(22) Date de dépôt: 24.05.2022
(51) Int. Cl.: G16H 40/67

(54) **PROCEDE D'ADAPTATION D'UN APPAREIL ORTHODONTIQUE**

(30) Priorité: 25.05.2021 FR 2105394
(62) Numéro(s) de document de la (des) demande(s) initiale(s) en application de l'article 76 CBE: 22730467.2 / 4 346 689
(71) Demandeur: Dental Monitoring, 75017 Paris (FR)
(72) Inventeur: PELLISSARD, Thomas, 75004 Paris (FR); SALAH, Philippe, 92200 Neuilly sur Seine (FR)
(74) Mandataire: Ipsilon

(57) **Abrégé**

L'invention concerne un système d'adaptation d'un appareil orthodontique destiné à un patient, ledit système comportant les étapes suivantes :
a) à un instant de paramétrage, détermination, par un premier professionnel de soins dentaires et spécifiquement pour ledit patient, de conditions pour une communication d'une information actualisée relative à la configuration de dentition du patient ;
b) à un instant actualisé postérieur à l'instant de paramétrage, acquisition, au moyen d'un appareil d'acquisition et par le patient ou un proche du patient, d'une représentation extraorale d'au moins une partie des arcades dentaires du patient, dite « représentation actualisée », ladite représentation actualisée étant constituée d'un ensemble d'images et/ou d'un modèle numérique tridimensionnel ;
c) analyse de ladite représentation actualisée, de manière à déterminer une information sur ladite configuration de dentition à l'instant actualisé, ou « information actualisée », l'information actualisée étant constituée d'un ensemble d'éléments d'information exprimant chacun un statut pour un objectif de traitement respectif, au regard de la représentation actualisée ;
d) communication de l'information actualisée à au moins un deuxième professionnel de soins dentaires, dans les conditions déterminées à l'étape a),
e) adaptation dudit appareil orthodontique en fonction de l'information actualisée communiquée à l'étape d).

## Description

### Domaine technique

La présente invention concerne un procédé d'adaptation d'un appareil orthodontique destiné à un patient, en particulier grâce à un suivi amélioré de la configuration de dentition du patient.

La « configuration de dentition » désigne
- la forme des mâchoires supérieure et inférieure, qui dépend notamment de la forme et la position des dents sur ces mâchoires, mais aussi de la forme générale de ces mâchoires, ainsi que
- l'agencement relatif de la mâchoire inférieure par rapport à la mâchoire supérieure, en particulier dans la position « bouche fermée ».

### Etat de la technique

Le demandeur a développé des procédés permettant le suivi à distance de la position de dents d'un patient, avant, pendant ou après un traitement orthodontique.

Ces procédés reposent sur la comparaison de photos prises par le patient, à un instant actualisé, au moyen de son téléphone portable, avec des vues d'un modèle numérique tridimensionnel d'une de ses arcades dentaires. Plus précisément, un modèle initial de l'arcade dentaire est réalisé à un instant initial, classiquement avec un scanner 3D, puis découpé en modèles de dent. Après que le patient a acquis les photos, le modèle initial est déformé, par déplacement des modèles de dent, pour correspondre au mieux aux photos. La comparaison des modèles initial et du modèle déformé ainsi obtenu fournit alors une information sur le déplacement des dents depuis l'instant initial. Le modèle initial étant très précis, il en est avantageusement de même pour le modèle déformé, et donc pour l'information résultant de ladite comparaison. Ces procédés sont notamment décrits dans PCT/EP2015/074868 ou PCT/EP2015/074859.

L'orthodontiste qui suit un patient peut ainsi désormais bénéficier de nombreuses données sur la position des dents du patient, sans que le patient ait à se déplacer. Typiquement, plusieurs centaines de données lui sont fournies. L'orthodontiste peut, à partir de ces données, évaluer si les objectifs de traitement sont remplis et, si nécessaire, adapter l'appareil orthodontique destiné au patient.

Il existe un besoin permanent pour faciliter et rendre plus précise cette adaptation de l'appareil orthodontique.

Un objectif de la présente invention est de répondre, au moins partiellement, à ce besoin.

### Résumé de l'invention

**Selon un premier aspect principal,** l'invention propose un procédé d'adaptation d'un appareil orthodontique destiné à un patient, ledit procédé comportant les étapes suivantes :
a) à un instant de paramétrage, paramétrage, par un premier professionnel de soins dentaires et spécifiquement pour ledit patient, de conditions pour une communication, par ordinateur, d'une information actualisée relative à la configuration de dentition du patient ;
b) à un instant actualisé postérieur à l'instant de paramétrage, acquisition, au moyen d'un appareil d'acquisition et par le patient ou un proche du patient, d'une représentation, de préférence extraorale, d'au moins une partie des arcades dentaires du patient, dite « représentation actualisée », ladite représentation actualisée étant de préférence constituée d'un ensemble d'images « actualisées » et/ou d'un modèle numérique tridimensionnel, ou « modèle actualisé » ;
c) analyse de ladite représentation actualisée, par ordinateur, de manière à déterminer une information sur ladite configuration de dentition à l'instant actualisé, ou « information actualisée », l'information actualisée étant de préférence constituée d'un ensemble d'éléments d'information exprimant chacun un statut déterminé au regard de la représentation actualisée, pour un objectif de traitement respectif, un statut pouvant en particulier indiquer que l'objectif de traitement est suivi et/ou est atteint et/ou n'est pas atteint à l'instant actualisé ;
d) communication, de préférence sur un écran, de l'information actualisée à au moins un deuxième professionnel de soins dentaires, dans les conditions déterminées à l'étape a),
e) de préférence, en fonction de l'information actualisée communiquée à l'étape d), adaptation ou non dudit appareil orthodontique.

Comme on le verra plus en détail dans la suite de la description, un procédé selon l'invention permet de filtrer l'information actualisée qui est présentée au deuxième professionnel de soins dentaires, en fonction de conditions qu'il a lui-même fixées pour la communication avec le patient. Les inventeurs ont découvert que la présentation d'un grand nombre de données pouvait s'avérer préjudiciable, en particulier pour l'analyse effectuée par le deuxième professionnel de soins dentaires, et donc pour l'efficacité, voire la précision de l'adaptation de l'appareil orthodontique effectuée en conséquence. Les inventeurs ont aussi observé que les professionnels de soins dentaires n'utilisent pas toujours les mêmes données et qu'un filtrage des données similaire pour tous les professionnels de soins dentaires pouvait donc être préjudiciable. Enfin, les inventeurs ont observé qu'un filtrage qui est identique pour tous les patients d'un professionnel de soins dentaires n'est pas satisfaisant non plus, chaque traitement orthodontique étant spécifique.

Selon l'invention, à l'instant de paramétrage, classiquement en début d'un traitement orthodontique, le premier professionnel de soins dentaires décide, spécifiquement pour le patient considéré et en fonction de sa propre appréciation de la situation dentaire du patient, dans quelles conditions l'information actualisée doit être diffusée. Avantageusement, à l'instant actualisé, le deuxième professionnel de soins dentaires, qui peut en particulier être le premier professionnel de soins dentaires, ne voit que les informations actualisées que le premier professionnel de soins dentaires a préalablement décidé de lui diffuser. Il peut ainsi se concentrer sur les seules informations qu'il estime utiles pour réaliser la meilleure analyse de la situation dentaire du patient à l'instant actualisé. Cette analyse est avantageusement plus rapide et plus précise car elle n'est pas perturbée par des informations que le premier professionnel de soins dentaires ne prendrait pas en considération.

Un procédé selon l'invention peut encore comprendre, notamment, une ou plusieurs des caractéristiques optionnelles suivantes :
- de préférence, les conditions déterminées à l'étape a) fixent pour au moins un dit objectif de traitement, de préférence pour chaque objectif de traitement, au moins une, voire une unique une plage temporelle, exclusivement pendant laquelle l'élément d'information correspondant, déterminé à l'étape c), est communicable à l'étape d), ou « plage de communication » ;
- les conditions déterminées à l'étape a) fixent pour au moins un dit objectif de traitement,
   - une durée minimale ou maximale entre l'instant actualisé et au moins un instant de référence, l'instant de référence étant de préférence un instant marquant la fin du traitement orthodontique ou d'une partie du traitement orthodontique ou l'instant de l'étape d) d'une série d'étapes b) à e) antérieure, et/ou
   - une fréquence maximale de communication de l'élément d'information ;
   - un ou plusieurs destinataires à qui l'élément d'information doit être communiqué ;
- les conditions déterminées à l'étape a) fixent pour ledit objectif de traitement, des dites plages de communication différentes en fonction du statut dudit objectif de traitement ;
- les conditions déterminées à l'étape a) fixent pour ledit objectif de traitement, une première dite plage de communication pour l'élément d'information exprimant que l'objectif de traitement est atteint, c'est-à-dire pour le statut « objectif atteint », et, immédiatement après ladite première plage de communication, une deuxième dite plage de communication pour l'élément d'information exprimant que l'objectif de traitement n'est pas atteint ;
- les conditions de communication d'au moins un élément d'information, de préférence de chaque élément d'information, déterminées à l'étape a), en particulier des plages de communication, sont en fonction de l'objectif de traitement concerné et/ou du statut de l'objectif de traitement exprimé par ledit élément d'information et/ou du destinataire de l'élément d'information ;
- le format et/ou le contenu d'un élément d'information sont en fonction du destinataire de l'élément d'information, par exemple sont différents selon que cet élément d'information est destiné à un orthodontiste, à un assistant de l'orthodontiste ou au patient ;
- de préférence, le statut d'un objectif de traitement, de préférence d'un objectif de traitement quelconque, est choisi dans un groupe de statuts potentiels comportant moins de 10 statuts potentiels ;
- de préférence, le nombre d'objectifs de traitement est inférieur à 30 ;
- de préférence, pour au moins un, de préférence pour chaque objectif de traitement, l'information actualisée comporte une évaluation du temps restant, à partir de l'instant actualisé, pour atteindre l'objectif de traitement, de préférence conformément à un plan de traitement établi avant l'instant actualisé, de préférence à l'instant de paramétrage, et/ou comporte une indication de la date depuis laquelle l'objectif de traitement est suivi ;
- de préférence, au moins un desdits objectifs de traitement est relatif au surplomb vertical ou au surplomb horizontal ;
- de préférence, l'appareil d'acquisition est un téléphone portable ;
- de préférence, la représentation actualisée est constituée d'un ensemble d'images, dites « images actualisées » ;
- à l'étape c), on soumet la représentation actualisée à au moins un réseau de neurones, de préférence convolutif ;
- de préférence, au moins un deuxième professionnel de soins dentaires est différent du premier professionnel de soins dentaires ;
- de préférence, le paramétrage à l'étape a) et la communication à l'étape d) sont réalisées au moyen d'une même interface d'ordinateur ;
- de préférence, l'interface présente simultanément sur un écran d'ordinateur :
   - un identifiant du patient ; et
   - une ligne chronologique représentant des instants successifs sélectionnables individuellement par un opérateur, notamment par les premier et deuxième professionnels de soins dentaires ; et
   - pour chaque instant sélectionnable sélectionné, des informations relatives à la configuration de dentition à l'instant sélectionné, de préférence au moins une représentation, de préférence extraorale, des arcades dentaires du patient à l'instant sélectionné, ou « représentation principale » et/ou un élément d'information exprimant un statut, à l'instant sélectionné, pour au moins un objectif de traitement et/ou au moins une observation relative à la configuration de dentition du patient à l'instant sélectionné ; et
   - une zone de suivi des objectifs de traitement fournissant des éléments d'information exprimant des statuts desdits objectifs de traitement à l'instant où l'interface est affichée ;
- à l'étape e), l'adaptation de l'appareil orthodontique comporte la fabrication ou la modification d'un appareil orthodontique destiné au patient ;
- à l'étape e), préalablement à l'adaptation de l'appareil orthodontique, un rendez-vous est pris entre le patient et le premier et/ou le deuxième professionnel de soins dentaire, en fonction de l'information actualisée ;
- l'appareil orthodontique adapté à l'étape e) est remis au patient, de préférence envoyé par la poste, en particulier lorsque l'appareil orthodontique est une gouttière orthodontique (« aligneur ») ;
- des séries d'étapes b) à e) sont répétées à différents instants actualisés pendant un traitement orthodontique d'un patient, les instants actualisés de deux séries successives étant de préférence séparés de plus d'une semaine, de plus de deux semaines et/ou de moins de 2 mois, de préférence de moins d'un mois.

A l'étape a), le paramétrage consiste à saisir dans un ordinateur les conditions de communication. L'étape c) et l'étape d) sont réalisées par ordinateur, de préférence sans assistance humaine, c'est-à-dire automatiquement. Il en est de même, de préférence, de l'adaptation de l'appareil orthodontique à l'étape e).

Les étapes a), c), et d), et de préférence e), sont mises en œuvre au moyen d'un ordinateur, de préférence réalisées par un ordinateur. Les ordinateurs utilisés pour ces étapes peuvent être identiques ou différents. S'ils sont différents, les ordinateurs sont en communication les uns avec les autres : en particulier, l'ordinateur paramétré à l'étape a) transfère les conditions de communication à l'ordinateur qui définit l'information actualisée à présenter à l'étape d) et à l'ordinateur qui communique l'information actualisée à l'étape d). L'ordinateur qui analyse ladite représentation actualisée à l'étape c) transmet l'information actualisée à l'ordinateur qui la communique à l'étape d). En outre, l'appareil d'acquisition transmet la représentation actualisée à l'ordinateur qui l'analyse à l'étape c).

L'invention concerne ainsi également :
- un programme d'ordinateur comprenant des instructions de code de programme pour mettre en œuvre les étapes a), c), d) et de préférence e) ;
- un support informatique sur lequel est enregistré un tel programme, par exemple une mémoire ou un CD-ROM, et
- un ordinateur dans lequel est chargé un tel programme.

L'invention concerne aussi un système pour la mise en œuvre d'un procédé selon l'invention, ledit système comportant :
- un premier ordinateur, paramétré, à l'étape a), par le premier professionnel de soins dentaires pour définir les conditions de communication de l'information actualisée ;
- un appareil d'acquisition, de préférence un téléphone portable, configuré pour acquérir ladite représentation actualisée à l'étape b) ;

- un deuxième ordinateur, identique ou différent du premier ordinateur apte à recevoir de l'appareil d'acquisition et à analyser ladite représentation actualisée pour déterminer l'information actualisée à l'étape c),
   apte à recevoir les conditions de communication du premier ordinateur, et
   apte à présenter l'information actualisée à un deuxième professionnel de soins dentaires selon les conditions de communication, à l'étape d) ;
- optionnellement, un troisième ordinateur configuré pour déterminer l'adaptation de l'appareil orthodontique à l'étape d), automatiquement ou, de préférence, avec l'assistance du deuxième professionnel de soins dentaires ;
- optionnellement, une machine de fabrication dudit appareil orthodontique ;
- optionnellement, un réseau de transport apte à transporter ledit appareil orthodontique depuis ladite machine de fabrication jusqu'au patient.

**Selon un deuxième aspect principal,** l'invention concerne également une interface d'ordinateur affichant, simultanément sur un écran d'ordinateur :
- un identifiant d'un patient ; et
- une ligne chronologique représentant des instants successifs sélectionnables individuellement par un opérateur ; et
- pour chaque instant sélectionnable sélectionné, des informations relatives à la configuration de dentition à l'instant sélectionné, de préférence au moins une représentation extraorale des arcades dentaires du patient à l'instant sélectionné, ou « représentation principale » et/ou un statut, à l'instant sélectionné, pour au moins un objectif de traitement et/ou au moins une observation relative à la configuration de dentition du patient à l'instant sélectionné ; et
- de préférence, une zone de suivi des objectifs de traitement fournissant des éléments d'information relatifs aux statuts desdits objectifs de traitement à l'instant où l'interface est affichée ;
- de préférence, un fil de discussion avec le patient.

L'invention concerne aussi un ordinateur programmé pour afficher une interface selon l'invention.

### Définitions

Par « patient », on entend toute personne malade ou non, subissant un traitement orthodontique. Sauf indication contraire, le « patient » est la personne pour laquelle on détermine les conditions de communication à l'étape a). Le patient est donc à distinguer des patients historiques qui concourent à l'établissement d'une base d'apprentissage.

Par « professionnel des soins dentaires », on entend un dentiste, un orthodontiste, un assistant paramédical, une société qui fabrique des traitements orthodontiques ou un laboratoire d'orthodontie.

Un « traitement orthodontique » est tout ou partie d'un traitement destiné à modifier la configuration de dentition d'un patient (traitement orthodontique actif) ou à la maintenir, en particulier après la fin d'un traitement orthodontique actif (traitement orthodontique passif).

Un traitement orthodontique est planifié avec un « plan de traitement ». On distingue ainsi le « traitement orthodontique » qui désigne la série d'opérations qui se déroule dans la réalité et le « plan de traitement », qui est le résultat de la conception du traitement orthodontique. Le plan de traitement est donc antérieur au traitement orthodontique.

Un traitement orthodontique peut être un traitement thérapeutique ou prophylactique, mais également à un traitement esthétique.

Un « appareil orthodontique » est un appareil porté ou destiné à être porté par un patient. Un appareil orthodontique peut être destiné à un traitement thérapeutique ou prophylactique, mais également à un traitement esthétique. Un appareil orthodontique peut être en particulier un appareil à arc et attaches, ou une gouttière orthodontique, ou un appareil auxiliaire du type Carrière Motion. Une telle gouttière s'étend de manière à suivre les dents successives de l'arcade sur laquelle elle est fixée. Elle définit une goulotte de forme générale en « U ». La configuration d'un appareil orthodontique peut être en particulier déterminée pour assurer sa fixation sur les dents, mais également en fonction d'un positionnement cible souhaité pour les dents. Plus précisément, la forme est déterminée de manière que, dans la position de service, l'appareil orthodontique exerce des forces tendant à déplacer les dents traitées vers leur positionnement cible (appareil orthodontique actif), ou à maintenir les dents dans ce positionnement cible (appareil orthodontique passif, ou « de contention »).

Une position ou une forme sont dites « anormales » lorsqu'elles ne sont pas conformes à une position ou à une forme, respectivement, attendues à l'instant considéré.

On appelle "téléphone portable" ou « téléphone mobile » un appareil de type iPhone^{®}. Un tel appareil pèse typiquement moins de 500 g, est doté d'un appareil photo comportant un objectif lui permettant de prendre des films ou des photos, voire d'un scanner lui permettant d'acquérir des modèles numériques tridimensionnels. Un téléphone portable est en outre capable d'échanger des données avec un autre appareil éloigné de plus de 500 km du téléphone portable, et est capable d'afficher lesdites données.

Par "image", on entend une représentation numérique en deux dimensions, comme une photographie ou une image extraite d'un film. Une image est formée de pixels.

Par « modèle », on entend un modèle tridimensionnel numérique. Un modèle est constitué d'un ensemble de voxels.

De manière générique, on appelle « représentation actualisée » un ensemble d'images « actualisées » ou un modèle « actualisé » représentant, au moins partiellement, les mâchoires du patient. Un ensemble d'images actualisées ou un modèle actualisé sont des exemples préférés de « représentations actualisées ».

Une image ou un modèle sont « extraoraux » lorsque l'appareil d'acquisition n'est pas introduit dans la bouche du patient lors de leur acquisition.

Une « représentation des arcades » du patient représente tout ou partie de ces arcades, de préférence plus de 2, plus de 5, plus de 8 dents, de préférence au moins une partie de chaque arcade, de préférence au moins une partie de la gencive de chaque arcade.

Une « angulation » est une orientation de l'axe optique d'un appareil d'acquisition par rapport à un patient, lors de l'acquisition d'une représentation actualisée, en particulier d'une image. Par extension, on dit qu'une image « présente » ou « a » une angulation ou est « associée à » une angulation lorsqu'elle a été acquise suivant cette angulation.

Un « écarteur » (« retractor » en anglais), ou « écarteur dentaire », est un dispositif destiné à retrousser les lèvres. Il comporte un rebord supérieur et un rebord inférieur, et/ou un rebord droit et un rebord gauche, s'étendant autour d'une ouverture d'écarteur et destinés à être introduits entre les dents et les lèvres. En position de service, les lèvres du patient sont en appui sur ces rebords, de sorte que les dents sont visibles à travers l'ouverture d'écarteur. Un écarteur permet ainsi d'observer les dents sans être gêné par les lèvres. Les dents ne reposent cependant pas sur l'écarteur, de sorte que le patient peut, en tournant la tête par rapport à l'écarteur, modifier les dents qui sont visibles à travers l'ouverture d'écarteur. Il peut aussi modifier l'écartement entre ses arcades dentaires. En particulier, un écarteur n'appuie pas sur les dents de manière à écarter les deux mâchoires l'une de l'autre. Dans un mode de réalisation, un écarteur est configuré de manière à écarter élastiquement l'une de l'autre les lèvres supérieure et inférieure de manière à dégager les dents visibles à travers l'ouverture d'écarteur. Des écarteurs sont par exemple décrits dans PCT/EP2015/074896, US 6,923,761, ou US 2004/0209225.

La position « bouche fermée » est la position d'occlusion dans laquelle les dentures des arcades supérieure et inférieure du patient sont en contact. Une position « bouche ouverte » est une position d'ouverture de la bouche, dans laquelle les dentures des arcades supérieure et inférieure du patient ne sont pas en contact.

Une « valeur actualisée » est une valeur pour un « attribut », ou « paramètre », de forme relatif à une configuration de dentition. Elle est déterminée à partir de la représentation actualisée, de préférence exclusivement à partir de la représentation actualisée, et dépend donc de la forme de l'ensemble formé par les mâchoires supérieure et inférieure du patient, dans au moins une position relative de la mâchoire inférieure par rapport à la mâchoire supérieure, par exemple en position bouche fermée, à l'instant actualisé. Une valeur actualisée est par exemple une mesure sur la représentation actualisée ou sur le résultat d'un traitement informatique de la représentation actualisée, ou est une valeur résultant de la soumission de la représentation actualisée à un réseau de neurones. Elle peut être déterminée directement sur la représentation actualisée, par exemple être une mesure d'un surplomb horizontal, ou indirectement, par exemple être le résultat d'une comparaison de la mesure d'un surplomb horizontal avec une valeur de référence, ou être un statut pour un objectif de traitement, par exemple être égale à « surplomb horizontal normal ». Une valeur actualisée peut être en particulier
- une dimension des arcades dentaires du patient,
- une position, relative ou dans un référentiel fixe par rapport au patient, d'un point des arcades dentaires du patient,
- une distance entre deux points des arcades dentaires du patient,
- une distance entre un ou plusieurs points des arcades dentaires du patient et un référentiel, ou
- une distance entre plusieurs points d'une même arcade dentaire, ou
- une combinaison d'une ou plusieurs desdites dimensions, positions et distances.

Un « élément d'information » exprime une valeur actualisée.

« Exprimer » une valeur actualisée, par exemple un statut, consiste à fournir, à un être humain, un enseignement relatif à cette valeur actualisée, par exemple fournir le statut pour un objectif de traitement, mais aussi présenter cette valeur actualisée, par exemple sous une forme graphique, ou sous la forme d'un symbole, ou d'un texte.

Un élément d'information peut être affiché sur un écran, par exemple un écran d'ordinateur ou de téléphone. Un élément d'information a donc un contenu qui découle de la valeur actualisée et un format qui définit la présentation de ce contenu (texte, police, élément graphique...).

Les « conditions de présentation » sont les règles qui définissent le format de chaque élément d'information. Les conditions de présentation peuvent en particulier définir le format de l'élément d'information en fonction de son destinataire et/ou de son contenu et/ou du média de communication utilisé. Par exemple, le format d'un élément d'information portant sur un surplomb vertical sera différent selon que l'élément d'information est adressé à l'orthodontiste ou au patient, selon que le surplomb vertical nécessite une intervention urgente de l'orthodontiste ou non, ou selon que l'élément d'information est adressé par mail ou par courrier. Les conditions de présentation sont de préférence définies et programmées avant l'étape a). Dans un mode de réalisation, elles peuvent être programmées ou modifiées par le premier professionnel de soins dentaires à l'étape a).

Les « conditions de communication » définissent, pour chaque élément d'information si, et de préférence à qui, ledit élément d'information doit être communiqué à l'étape d). Elles peuvent dépendre de l'instant actualisé et/ou de la valeur actualisée associée à l'élément d'information et/ou de l'âge du patient et/ou du traitement orthodontique ou du type de traitement orthodontique suivi par le patient, par exemple avec des gouttières orthodontiques ou avec un appareil de type arc et attaches ou mixte, et/ou du statut d'un ou plusieurs autres objectifs de traitement. Elles sont définies à l'étape a).

Un « objectif de traitement » est un objectif pour la configuration de dentition du patient, pour la fin d'un traitement orthodontique actif ou pour une étape intermédiaire (jalon) d'un traitement orthodontique actif. Il est classiquement fixé par un orthodontiste. Pour un traitement orthodontique passif, un objectif de traitement est une absence de modification de la configuration de dentition depuis une date antérieure.

L'attribut d'un objectif de traitement est appelé « statut ». Un « statut » ne peut prendre qu'un nombre limité de valeurs, dites « statuts potentiels ». A un instant donné, le statut est égal à un des statuts potentiels pour l'objectif de traitement, qui constitue alors une « valeur actualisée ». Avantageusement, un élément d'information exprimant un statut est facile à appréhender, ce qui facilite la prise de décision après l'étape d).

Un objectif de traitement est « suivi » lorsqu'un élément d'information est déterminé à l'étape c) pour cet objectif de traitement et peut faire l'objet d'une communication à l'étape d), selon les conditions de communication. La donnée selon laquelle l'objectif de traitement est suivi ou non n'apporte pas d'information sur la configuration de dentition du patient à l'instant actualisé et n'est donc pas une « valeur actualisée ».

« L'information actualisée » est constituée d'un ensemble d'éléments d'information. L'information est dite « actualisée » car elle est déterminée à partir de la représentation actualisée acquise à l'instant actualisé.

Les procédés selon l'invention sont mis en œuvre par ordinateur, de préférence exclusivement par ordinateur, hors l'acquisition de la représentation actualisée et la fabrication des appareils orthodontiques. Par « ordinateur », on entend tout appareil électronique, ce qui inclut un ensemble de plusieurs machines, ayant des capacités de traitement informatique. L'ordinateur peut être un serveur à distance du patient, par exemple être le « cloud ». De préférence, l'ordinateur est un téléphone portable.

Classiquement, un ordinateur comporte en particulier un processeur, une mémoire, une interface homme-machine, comportant classiquement un écran, un module de communication par internet, par WIFI, par Bluetooth^{®} ou par le réseau téléphonique. Un logiciel configuré pour mettre en œuvre un procédé de l'invention ou une étape d'un tel procédé est chargé dans la mémoire de l'ordinateur. L'ordinateur peut être également connecté à une imprimante.

« Premier », « deuxième », « actualisé », sont utilisés à des fins de clarté.

« Antérieur » et « postérieur » font référence des instants qui se succèdent dans le temps.

« Vertical », « horizontal », « droite », « gauche », « devant » ou « de face », « derrière », « au-dessus », « au-dessous » font référence à un patient qui se tient debout, verticalement.

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- [Fig 1] la figure 1 représente, schématiquement, un procédé d'analyse selon l'invention, dans un mode de réalisation préféré ;
- [Fig 2] la figure 2 représente, schématiquement, une interface selon l'invention ;
- [Fig 3] la figure 3 représente un exemple de zone de suivi des objectifs de traitement ;
- [Fig 4] la figure 4 représente un exemple de boîte de dialogue pour paramétrer un objectif de traitement ;
- [Fig 5] la figure 5 illustre le suivi d'un objectif de traitement, dans un mode de réalisation préféré de l'invention ;
- [Fig 6] la figure 6 représente un exemple d'image actualisée, prise de face ;
- [Fig 7] la figure 7 représente un exemple de système selon l'invention ;
- [Fig 8] la figure 8 représente un exemple de dispositif d'acquisition comportant un téléphone portable et un embout buccal.

Dans les différentes figures, des références identiques sont utilisées pour désigner des objets analogues ou identiques.

### Description détaillée

Un but de l'invention est de faciliter l'analyse de la configuration de dentition du patient, en particulier pour déterminer les actions à entreprendre en conséquence, et en particulier adapter un appareil orthodontique destiné à un patient.

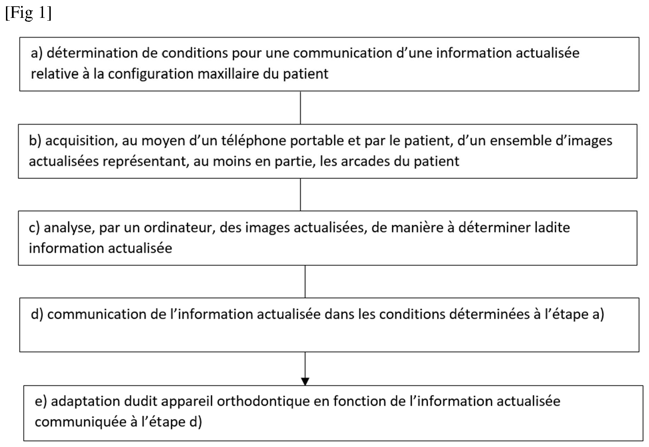

Un procédé selon l'invention est illustré sur la figure 1. Ce procédé est décrit avec, comme représentation actualisée, un ensemble d'images actualisées. Cependant, à l'étape b), en alternative ou en complément des images actualisées, il est possible d'acquérir, avec un scanner portable, de préférence intégré dans le téléphone portable du patient, un modèle des arcades dentaires du patient, dit « modèle actualisé ». L'analyse à l'étape c) est adaptée en conséquence.

**A l'étape a)**, à un instant dit « instant de paramétrage », un premier professionnel de soins dentaires, de préférence un orthodontiste, détermine, spécifiquement pour le patient, des conditions pour une communication d'une information actualisée relative à la configuration de dentition du patient. Puis ces conditions de communication sont saisies dans un ordinateur.

Les conditions de communication déterminées à l'étape a) peuvent être choisies dans une base de données informatique contenant plusieurs ensembles prédéterminés de conditions de communication, par exemple plus de 2 plus de 5 ou plus de 10, et/ou moins de 500 ensembles prédéterminés. Le premier professionnel de soins dentaires peut optionnellement modifier les conditions de l'ensemble prédéterminé qu'il a choisi.

Les conditions de communication déterminées à l'étape a) peuvent être également entièrement définies par le premier professionnel de soins dentaires.

L'instant de paramétrage peut être en particulier avant un traitement orthodontique ou avant une étape intermédiaire d'un traitement orthodontique, de préférence moins de 2 semaines, de préférence moins d'une semaine avant ledit traitement orthodontique ou ladite étape intermédiaire, respectivement. L'instant de paramétrage peut être par exemple un instant prévu pour un changement de gouttière orthodontique ou pour une modification d'un traitement avec un appareil orthodontique à arc et attache. L'instant de paramétrage peut être également lors d'une étape d) d'une série d'étapes b) à d) antérieure à l'instant actualisé.

Le premier professionnel de soins dentaires est de préférence l'orthodontiste ou le dentiste qui suit un traitement orthodontique qu'il a prescrit au patient.

De préférence, au moins un élément d'information exprime un statut pour un objectif de traitement.

Les conditions de communication peuvent en particulier contenir une ou plusieurs :
- conditions temporelles pour que l'élément d'information soit communiqué, en particulier une ou plusieurs plages de communication, et/ou
   une durée minimale ou maximale entre l'instant actualisé et au moins un instant de référence, par exemple un instant marquant la fin du traitement orthodontique ou d'une partie du traitement orthodontique, ou l'instant de l'étape d) d'une série d'étapes b) à e) antérieure comprenant optionnellement une étape a), et/ou
   une fréquence maximale de communication de l'élément d'information, de préférence en fonction du destinataire de l'élément d'information ; et/ou
- conditions sur le ou les destinataires de l'élément d'information, en particulier pour fixer à qui l'élément d'information doit être communiqué.

De préférence, lorsqu'une condition concerne un objectif de traitement, elle fait intervenir le statut de l'objectif de traitement à l'instant actualisé, c'est-à-dire que l'application de la condition à l'élément d'information relatif audit objectif de traitement aboutit à un résultat différent selon le statut de l'objectif de traitement à l'instant actualisé.

A l'étape a), les conditions pour la communication déterminent de préférence, pour au moins un, de préférence pour chaque élément d'information, au moins une plage de communication à laquelle l'instant actualisé doit appartenir pour que ledit élément d'information soit communiqué au deuxième professionnel de soins dentaires à l'étape d). Avantageusement, il est ainsi possible, à l'instant de paramétrage, de fixer les plages temporelles auxquelles les éléments d'informations devront être communiqués.

De préférence, la plage de communication associée à un élément d'information est définie en fonction du destinataire à qui ledit élément d'information est destiné.

De préférence, une plage de communication, de préférence une plage de communication quelconque, a une durée limitée, de préférence inférieure à 3 mois, de préférence inférieure à 2 mois et/ou supérieure à 1 semaine.

Une plage de communication, de préférence une plage de communication quelconque, commence de préférence après l'instant de paramétrage, de préférence plus d'une semaine après l'instant de paramétrage.

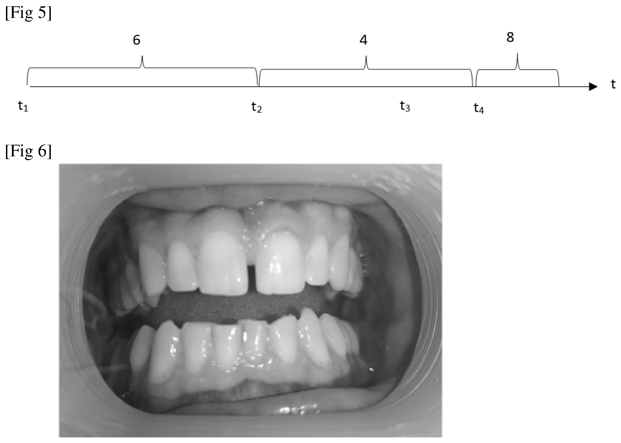

La figure 5 illustre le suivi d'un objectif de traitement dans un mode de réalisation préféré. Sur un axe représentant le temps t, on distingue une dite plage de communication 4, une période 6 antérieure à la plage de communication 4 et une période 8 postérieure à la plage de communication 4.

Le suivi de l'objectif commence à l'instant de paramétrage t₁. Le statut de l'objectif de traitement ne devient communicable au deuxième professionnel de soins dentaires qu'à partir de l'instant t₂ et seulement jusqu'à l'instant t₄. Dans ce mode de réalisation préféré, si le statut indique, pendant la plage de communication, que l'objectif de traitement n'est pas atteint, il n'est pas communiqué. De préférence, s'il n'est pas atteint à l'instant t₄, il est cependant communiqué à l'instant t₄ ou à la première étape d) qui suit l'instant t₄. S'il est atteint pendant la plage de communication 4, à l'instant t₃, il est communiqué à cet instant t₃.

L'intervalle [t₁-t₂] peut être nul dans le cas où le deuxième professionnel de soins dentaires doit être notifié du statut de l'objectif de traitement dès le paramétrage.

Lorsque l'élément d'information est un statut pour un objectif de traitement, le début de la plage de communication peut être en particulier le moment auquel le premier professionnel de soins dentaires estime que l'objectif de traitement sera atteint. La fin de la plage de communication peut être en particulier le moment auquel le premier professionnel de soins dentaires souhaite être averti si l'objectif de traitement n'est pas atteint, par exemple pour contacter le patient, par exemple pour lui communiquer une information et/ou une recommandation et/ou une nouvelle instruction dans le cadre du traitement orthodontique, et/ou prendre un rendez-vous avec lui.

Dans un mode de réalisation, à l'étape a), les conditions pour la communication déterminent de préférence, pour au moins un, de préférence pour chaque élément d'information de l'information actualisée, une fréquence maximale de communication à l'étape d), et/ou un intervalle de temps minimal depuis la mise en œuvre du procédé précédente. Avantageusement, il est ainsi possible d'éviter de communiquer à une fréquence élevée un élément d'information dont l'évolution est lente.

Dans un mode de réalisation préféré, à l'étape a), les conditions pour la communication déterminent pour au moins un, de préférence pour chaque élément d'information de l'information actualisée, le ou les deuxièmes professionnels de soins dentaires à qui l'élément d'information doit être communiqué. Un élément d'information peut être ainsi transmis, par exemple, exclusivement à un orthodontiste, ou exclusivement à un assistant de l'orthodontiste, ou à la fois à l'orthodontiste et à l'assistant. Avantageusement, l'orthodontiste peut ainsi décider qu'un élément d'information qui peut être analysé par un assistant, par exemple parce qu'il ne conduit pas à une modification du traitement orthodontique, n'a pas besoin de lui être communiquée. Il peut aussi décider qu'un élément d'information ne sera communiqué qu'en fonction de sa valeur et/ou de l'instant actualisé. Un procédé selon l'invention permet ainsi de distribuer les éléments d'information aux seules personnes qui, à un instant actualisé, ont potentiellement une action à réaliser pour le patient, par exemple pour modifier le traitement orthodontique, contacter le patient, préparer un nouvel appareil orthodontique ou organiser un rendez-vous.

En particulier, pour un objectif de traitement, le choix du deuxième professionnel de soins dentaires peut dépendre de l'objectif de traitement concerné et/ou du statut de l'objectif de traitement concerné à l'instant actualisé.

Dans un mode de réalisation préféré, à l'étape a), les conditions pour la communication déterminent pour au moins un, de préférence pour chaque élément d'information de l'information actualisée, si l'élément d'information doit être communiqué au patient, en particulier sur son téléphone portable, alternativement ou, de préférence, en complément à la communication au deuxième professionnel de soins dentaires.

Dans un mode de réalisation préféré, à l'étape a), les conditions pour la communication déterminent pour au moins un, de préférence pour chaque objectif de traitement, si l'objectif de traitement est suivi ou non.

Les conditions déterminées à l'étape a) permettent un filtrage fin de l'information actualisée, en fonction de sa nature et/ou de sa valeur et/ou de l'instant actualisé. Chaque destinataire, et en particulier chaque deuxième professionnel de soins dentaires ne reçoit ainsi que les éléments d'information qui le concernent à l'instant actualisé. L'efficacité de la communication, et donc l'efficacité du traitement, en sont améliorées.

L'élément d'information peut en particulier exprimer un statut pour un objectif de traitement.

Les conditions de communication peuvent par exemple disposer que :
- l'élément d'information n'est communiqué que si l'objectif de traitement est atteint ou n'est pas atteint à l'instant actualisé ; et/ou
- l'élément d'information n'est communiqué que pendant une plage de communication prédéterminée ; et/ou
- l'élément d'information n'est communiqué que s'il n'a pas été communiqué depuis moins de J jours ; et/ou
- l'élément d'information est communiqué au patient et/ou à un deuxième professionnel de soins dentaires, systématiquement ou en fonction de la plage de communication et/ou en fonction de sa valeur ; et/ou
- l'élément d'information est systématiquement communiqué si l'instant actualisé est postérieur à une plage de communication prédéterminée ou si l'instant actualisé est séparé de la fin d'une plage de communication par une période d'une durée inférieure à un nombre prédéterminé de jours.

**A l'étape b)**, on acquiert, à l'instant actualisé, au moyen d'un appareil d'acquisition, la représentation actualisée, de préférence un ensemble d'images actualisées, représentant les arcades dentaires du patient.

L'instant actualisé peut être
- indépendant de tout traitement orthodontique, par exemple pour que chacun puisse contrôler sa configuration de dentition à tout instant, avec son téléphone portable ;
- pendant un traitement orthodontique actif ;
- après un traitement orthodontique actif, en particulier lors d'un traitement orthodontique passif.

Le procédé d'analyse peut notamment être mis en œuvre pendant un traitement orthodontique actif pour en contrôler le déroulement, l'instant actualisé étant de préférence moins de 3 mois, moins de 2 mois, et/ou plus de 4 jours, plus d'une semaine, de préférence plus de 2 semaines après la pose d'un appareil orthodontique actif, par exemple d'une gouttière orthodontique (ou « aligneur ») ou d'un arc orthodontique, destiné à corriger le positionnement des dents du patient.

Le procédé d'analyse peut être également mis en œuvre après un traitement orthodontique, pour vérifier que le positionnement des dents n'évolue pas de façon défavorable (« récidive »). L'instant actualisé est alors de préférence moins de 6 mois, moins de 3 mois, et/ou plus de 1 semaine, de préférence plus de 2 semaines après la fin du traitement orthodontique actif et la pose d'un appareil orthodontique passif destiné maintenir les dents en position, dit « attèle de contention ».

L'appareil d'acquisition est de préférence choisi parmi un téléphone portable, un appareil photo dit « connecté », une montre dite « intelligente », ou « smartwatch », une tablette ou un ordinateur personnel, fixe ou portable, comportant un système d'acquisition d'images, comme une webcam ou un appareil photo. De préférence, l'appareil d'acquisition est un téléphone portable. De préférence, l'appareil d'acquisition, en particulier le téléphone portable, n'est pourvu d'aucune optique spécifique pour l'acquisition des images actualisées.

De préférence encore, pour acquérir une image actualisée, l'appareil d'acquisition des images actualisées est écarté de la bouche du patient de de plus de 5 cm, plus de 8 cm, voire plus de 10 cm et/ou inférieure à 50 cm. Cette distance n'a avantageusement pas besoin d'être fixée précisément.

L'appareil d'acquisition est utilisé, généralement à distance de tout professionnel de soins dentaires, par un opérateur qui est le patient ou un proche du patient. De préférence, l'acquisition des images actualisées est effectuée par le patient.

De préférence, l'acquisition est effectuée sans utilisation d'un support, prenant appui sur le sol et immobilisant l'appareil d'acquisition, et notamment sans trépied.

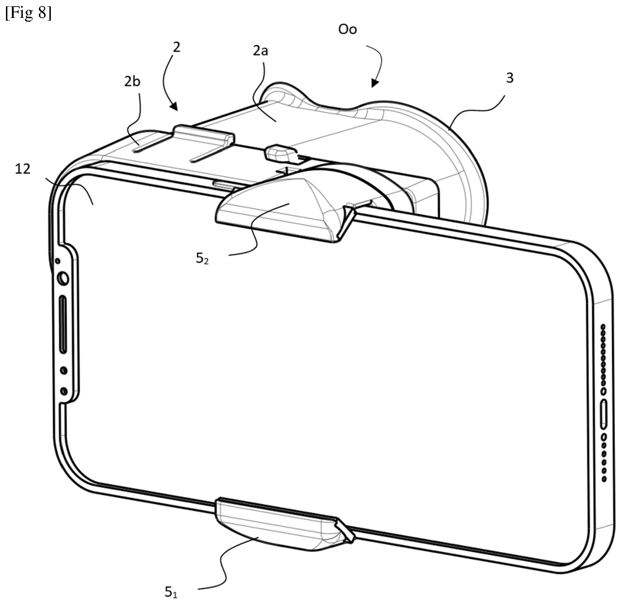

Dans un mode de réalisation cependant, l'appareil d'acquisition est fixé sur un support qui est positionné en appui sur le corps du patient, de préférence un embout buccal 2 partiellement introduit dans la bouche du patient, comme illustré sur la figure 8.

De préférence, l'embout buccal 2 comporte :
- un espaceur tubulaire 2a qui définit l'ouverture orale Oo et comporte de préférence un rebord 3 s'étendant radialement vers l'extérieur, à la périphérie de l'ouverture orale Oo, destiné à être introduit entre les lèvres et les dents du patient, et
- un adaptateur 2b sur lequel le téléphone portable 12 est fixé, par exemple serré entre deux mâchoires 5₁ et 5₂, comme illustré sur la figure 8, l'adaptateur 2b étant fixé rigidement sur l'espaceur tubulaire 2a, de préférence de manière amovible, par exemple au moyen d'un clips, ou venu de matière avec l'espaceur, de manière que l'objectif du téléphone portable puisse « voir » l'ouverture orale.

Lorsque le support est rigide, il impose avantageusement une distance prédéterminée entre l'appareil d'acquisition et la bouche du patient. L'analyse des images actualisées à l'étape c) en est facilitée.

Dans un mode de réalisation, le support porte un écarteur dentaire conventionnel.

Un exemple de support est décrit dans la demande de brevet européen déposée le 10 octobre 2017 sous le n° 17 306361.1.

La représentation actualisée, en particulier les images actualisées, sont de préférence extra-orales, c'est-à-dire que l'appareil d'acquisition n'est pas introduit dans la bouche du patient.

De préférence, les images actualisées sont des photographies ou des images extraites d'un film. Elles sont de préférence en couleurs, de préférence en couleurs réelles. De préférence, elles représentent les arcades dentaires sensiblement comme les voit l'opérateur de l'appareil d'acquisition. La figure 6 représente un exemple d'un telle image actualisée, prise de face.

Dans un mode de réalisation, le patient porte un écarteur dentaire pour mieux découvrir ses dents. De préférence cependant, aucun écarteur dentaire n'est utilisé. De préférence, le patient ne porte pas d'écarteur dentaire à l'étape b). Bien entendu, si nécessaire, le patient peut devoir écarter une joue ou une lèvre avec un doigt ou avec une cuillère ou tout autre ustensile adapté à cet effet, par exemple.

La représentation actualisée peut représenter les arcades en position « bouche ouverte » ou « bouche fermée ». De préférence, au moins une image actualisée est acquise alors que le patient a la bouche ouverte. De préférence, au moins une image actualisée est acquise alors que le patient a la bouche fermée.

Une image actualisée, de préférence chaque image actualisée représente une ou plusieurs dents et au moins une partie de la gencive, voire des lèvres ou du nez du patient.

De préférence encore, à l'étape b), on acquiert plusieurs images actualisées prises selon des angulations différentes, c'est-à-dire avec des orientations différentes de l'appareil d'acquisition par rapport à la cavité buccale du patient. Par exemple, l'ensemble d'images actualisées peut comporter 6 images représentant les arcades dentaires « vues de face », « vues de face-droite », « vues de droite », « vues de face-gauche », « vues de gauche » et « vues de dessous », respectivement.

De préférence, au moins une image actualisée est acquise face au patient (vue de face).

De préférence, au moins une image actualisée est acquise depuis la droite du patient, et au moins une image actualisée est acquise depuis la gauche du patient.

De préférence, l'opérateur est guidé lors de l'étape b), de préférence en temps réel, pour orienter l'appareil d'acquisition suivant des angulations prédéterminées, et/ou, de préférence, pour prendre un nombre d'images prédéterminé selon les différentes angulations, et/ou pour orienter l'appareil d'acquisition selon les angulations requises. A cet effet, un applicatif est de préférence chargé dans l'appareil d'acquisition afin d'assurer un contrôle embarqué lors de l'étape b), c'est-à-dire de vérifier que le nombre et/ou l'angulation et/ou la qualité des images actualisées sont satisfaisants.

L'ensemble d'images actualisées obtenu à l'issue de l'étape a) comporte de préférence plus de deux, de préférence plus de trois, de préférence plus de 5, de préférence plus de 6 et/ou moins de 30, de préférence moins de 20, de préférence moins de 15, de préférence moins de 10 images actualisées.

**A l'étape c),** un ordinateur analyse la représentation actualisée, de préférence des images actualisées, et détermine l'information actualisée.

Les méthodes connues d'analyse d'images peuvent être utilisées pour déterminer une valeur « actualisée » pour un attribut relatif à la configuration de dentition du patient à l'instant actualisé, puis générer un élément d'information exprimant cette valeur « actualisée ».

De préférence, on soumet l'ensemble d'images actualisées à un réseau de neurones entrainé pour fournir une telle valeur « actualisée ».

L'attribut peut être quantitatif, par exemple définir
- une position d'un ou plusieurs points d'une même arcade ou des deux arcades dentaires du patient,
- une dimension ou une distance entre plusieurs points d'une même arcade, par exemple une largeur d'arcade, un écartement entre deux dents,
- une distance entre plusieurs points des deux arcades, par exemple pour la détermination d'une classe d'occlusion ou la déviation des milieux inter-incisifs,
- une évolution dans le temps d'une dite dimension, d'une dite distance et/ou d'une dite position listée(s) ci-dessus, par exemple une différence entre les positions d'un même point des arcades à des instants différents, ou une vitesse de déplacement dudit point entre lesdits instants, ou une accélération de déplacement dudit point entre lesdits instants.

L'attribut peut être synthétique, et en particulier être une synthèse d'un ou plusieurs des exemples d'attributs listés ci-dessus. Par exemple, il peut définir un niveau d'encombrement, un statut pour un objectif de traitement, une évolution « à la hausse », « à la baisse » ou « stable », par exemple de l'activité d'un arc orthodontique et/ou d'un déplacement d'une ou plusieurs dents, ou définir un score résultant d'une combinaison de plusieurs autres attributs, par exemple pour évaluer la complexité de la situation dentaire du patient.

Le réseau de neurones est de préférence spécialisé dans la classification d'images. De préférence, le réseau de neurones est convolutif (CNN, en anglais « Convolutional neural network »), de préférence choisi parmi les réseaux de neurones suivants : AlexNet (2012), ZF Net (2013), VGG Net (2014), GoogleNet (2015), Microsoft ResNet (2015), Caffe : BAIR Reference CaffeNet, BAIR AlexNet, Torch :VGG_CNN_S, VGG_CNN_M, VGG_CNN_M_2048, VGG_CNN_M_1024, VGG_CNN_M_128, VGG_CNN_F, VGG ILSVRC-2014 16-layer, VGG ILSVRC-2014 19-layer, Network-in-Network (Imagenet & CIFAR-10), Google : Inception (V3, V4).

De préférence, un bloc de traitement « squeeze-and-excitation (SE) », tel que décrit par Jie Hu et al, dans « Squeeze-and-Excitation Networks », arXiv:1709.01507v4 [cs.CV] 16 May 2019, est ajouté à un opérateur convolutif CNN. De préférence encore, le réseau de neurones est du type VGG avec un bloc SE.

Pour être opérationnel, le réseau de neurones doit être classiquement entrainé par un processus d'apprentissage appelé *« deep learning »*, à partir d'une base d'apprentissage historique adaptée à la fonction souhaitée.

Classiquement, on crée une base d'apprentissage historique, puis on entraine le réseau de neurones avec cette base.

### Base d'apprentissage historique

L'entrainement d'un réseau de neurones est un processus bien connu de l'homme de l'art. Il consiste à confronter le réseau de neurones à une base d'apprentissage historique contenant des enregistrements historiques contenant chacun une donnée d'entrée et une donnée de sortie.

Le réseau de neurones apprend ainsi à faire « correspondre », c'est-à-dire à connecter l'une à l'autre, les données d'entrée et de sortie.

Pour que le réseau de neurones apprenne à évaluer, à partir d'un ensemble d'images actualisées, une valeur pour l'attribut relatif à la configuration de dentition, la base d'apprentissage historique est de préférence constituée d'un ensemble d'enregistrements historiques comportant chacun :
- un ensemble d'images « historiques » représentant toutes les arcades dentaires « historiques » d'un patient « historique », de préférence des photos ;
- un descriptif historique de l'ensemble d'images historiques, le descriptif comportant une valeur « historique » pour ledit attribut, relativement à la configuration de dentition des arcades représentées sur les images historiques.

De préférence, la base d'apprentissage historique comporte plus de 1000, plus de 5 000, de préférence plus de 10 000, de préférence plus de 30 000, de préférence plus de 50 000, de préférence plus de 100 000 enregistrements historiques. Plus le nombre d'enregistrements est élevé, meilleure est la capacité d'analyse du réseau de neurones. Le nombre d'enregistrements historiques est classiquement inférieur à 10 000 000 ou à 1 000 000.

Les enregistrements historiques sont chacun associés à un patient historique respectif.

### Images historiques

Un ensemble d'images historiques est de préférence similaire à l'ensemble d'images actualisées.

De préférence, les ensembles d'images historiques comportent tous le même nombre d'images historiques, quel que soit l'enregistrement historique considéré. Ce nombre est de préférence supérieur à 1, à 2, à 4, à 5 et/ou inférieur à 100, à 50, à 20, à 15 ou à 10, de préférence compris entre 5 et 15.

Dans un mode de réalisation, ce nombre est de 3, les 3 images historiques ayant des angulations différentes.

Les images historiques d'un ensemble d'images historiques sont de préférence acquises dans des conditions similaires aux images actualisées acquises à l'étape b). Autrement dit, l'ensemble des images actualisées doit être tel qu'il aurait pu être utilisé pour un enregistrement historique.

Le nombre d'images historiques d'un enregistrement historique est de préférence identique au nombre d'images actualisées de l'ensemble d'images actualisées de l'étape a).

De préférence, les images historiques sont
- acquises avec un appareil d'acquisition du même type que celui utilisé pour les images actualisées, de préférence un téléphone portable si les images actualisées sont acquises avec un téléphone portable ;
- de préférence acquises avec sensiblement les mêmes angulations que les images actualisées ;
- de préférence des photos en couleurs, de préférence en couleurs réelles si les images actualisées sont des photos en couleur, respectivement en couleurs réelles ;
- extra-orales, si les images actualisées sont extra-orales ;
- de préférence prises avec une orientation et une distance de l'appareil d'acquisition par rapport à la bouche sensiblement identiques à celles lors de l'acquisition des images actualisées ;
- de préférence acquises alors que le patient historique porte un écarteur dentaire si c'est le cas lors de l'acquisition des images actualisées ;
- de préférence acquises alors que l'appareil d'acquisition est fixé sur un support qui est positionné en appui sur le corps du patient historique, de préférence introduit dans la bouche du patient historique, si c'est le cas pour l'acquisition des images actualisées.

### Descriptif historique

Un descriptif historique d'un ensemble d'images historiques comporte une valeur pour ledit attribut relatif à la configuration de dentition du patient historique.

L'attribut est le même pour toutes les images historiques de l'ensemble. De préférence, le réseau de neurones est spécialisé pour un attribut, c'est-à-dire que l'attribut est le même pour tous les enregistrements historiques.

La détermination de la valeur d'un descriptif historique ne pose pas de difficulté. Elle peut être déterminée par tout moyen, par exemple manuellement ou par ordinateur, notamment en effectuant des mesures sur le patient historique ou sur un moulage en plâtre de ses dents ou sur un modèle tridimensionnel numérique de l'arcade dentaire portant la dent considérée.

### Entrainement

Le réseau de neurones est ensuite entrainé avec la base d'apprentissage historique, en lui présentant successivement les enregistrements historiques, et plus précisément, pour chaque enregistrement historique, les ensembles d'images historiques en entrée et les valeurs de l'attribut du descriptif historique en sortie.

Il apprend ainsi à fournir, à sa sortie, à partir d'un ensemble d'images similaire à un ensemble d'images historiques qu'on lui présente en entrée, une valeur pour l'attribut. En particulier, après avoir été ainsi entrainé, le réseau de neurones peut, lorsqu'on lui présente en entrée un ensemble d'images actualisées, fournir en sortie une valeur « actualisée » pour l'attribut, et ainsi renseigner sur la configuration de dentition des arcades représentées sur les images actualisées.

Après l'entrainement, l'ensemble des images actualisées constitué à l'étape b) est entré dans le réseau de neurones. Le réseau de neurones fournit en réponse une valeur pour l'attribut, dite « valeur actualisée ».

Dans un mode de réalisation préféré, plusieurs réseaux de neurones spécialisés chacun pour un attribut sont entrainés. De préférence, on acquiert alors à l'étape b) un nombre d'images actualisées suffisant pour constituer des ensembles d'images actualisées « spécialisés » adaptés pour chacun des réseaux de neurones spécialisés.

Le réseau de neurones peut utiliser un modèle d'arcades dentaires à la place d'un ensemble d'images. L'entrainement du réseau de neurones est similaire à celui décrit ci-dessus, les ensembles d'images historiques et actualisées étant remplacées par des modèles historiques et actualisé, respectivement.

Avant de préparer un élément d'information associé à une valeur actualisée, la valeur actualisée peut être traitée, par exemple remplacée par le résultat de la différence entre la valeur actualisée et une valeur de référence, ou être combinée avec d'autres valeurs actualisées. Par souci de clarté, on appelle valeur actualisée « dérivée » le résultat d'un tel traitement.

Pour le suivi d'un traitement orthodontique, la valeur de référence peut être la valeur attendue pour l'attribut à l'instant actualisé ou à la fin du traitement orthodontique, avec une marge de tolérance éventuelle. En dehors de tout traitement, une valeur de référence peut être par exemple une valeur considérée comme normale pour l'attribut, par exemple à l'instant actualisé.

Dans un mode de réalisation, la valeur de référence est indépendante du patient, c'est-à-dire applicable à tout patient d'un groupe de patients. Elle constitue ainsi un étalon. De préférence, l'étalon est spécifique à une pathologie et/ou à un type de traitement orthodontique, et/ou à un groupe de patients partageant une caractéristique commune, par exemple appartenant à une même classe d'âge et/ou de même sexe. L'étalon peut en particulier définir une configuration de dentition en fin d'un traitement ou à l'instant actualisé.

Une valeur actualisée, optionnellement dérivée, est présentée, à l'étape d), dans les conditions de communication, sous la forme d'un élément d'information correspondant. A l'étape c), on détermine, par ordinateur et suivant des conditions de représentation, l'élément d'information à partir de la valeur actualisée, optionnellement dérivée.

Dans un mode de réalisation, à l'étape c), l'information actualisée résulte de la seule analyse des images actualisées, éventuellement à l'aide d'une ou plusieurs valeurs de référence.

Dans un mode de réalisation, un élément d'information est en fonction du destinataire à qui il est destiné et/ou de la valeur actualisée.

Par exemple, si la valeur actualisée indique que le traitement orthodontique ne se déroule pas correctement, l'élément d'information correspondant destiné au patient peut être une phrase rappelant la nécessité de suivre les prescriptions de l'orthodontiste ou lui demandant de prendre un rendez-vous. L'élément d'information correspondant destiné à un assistant de l'orthodontiste peut être un bon de commande pour commander une nouvelle gouttière orthodontique ou un nouvel arc dentaire. L'élément d'information correspondant destiné à l'orthodontiste peut être une donnée médicale. Si la valeur actualisée indique que le traitement orthodontique ne se déroule pas correctement, l'élément d'information peut être différent selon la gravité de la situation. Par exemple, si la situation est grave, l'élément d'information destiné au patient peut être une phrase en rouge indiquant avec instance la nécessité de prendre rendez-vous très rapidement. Si la situation n'est pas grave, l'élément d'information destiné au patient peut être une phrase en noir indiquant, pour information, que le traitement dévie légèrement du plan de traitement.

Bien entendu, si la connaissance des conditions de communication est nécessaire pour préparer un élément d'information, l'ordinateur qui prépare les éléments d'information est informé desdites conditions de communication. Par exemple, si le contenu et/ou le format d'un élément d'information dépendent de son destinataire, ces conditions permettent de savoir quels sont les destinataires de la communication à l'étape d), par exemple en fonction de la valeur actualisée et/ou de l'instant actualisé, et donc de préparer l'élément d'information en conséquence.

Dans un mode de réalisation, une information actualisée prend la forme d'un message adapté à son destinataire. De préférence, le message est généré par ordinateur, de préférence choisi dans une base de messages type, de préférence adaptée au destinataire. Dans un mode de réalisation, il est vérifié, et éventuellement modifié par un professionnel de soins dentaires avant d'être présente à l'étape d).

En particulier, si un élément d'information doit être communiqué au patient, de préférence sur son téléphone portable, il présente de préférence la forme d'un message spécifiquement adapté au patient. La communication de l'élément d'information est ainsi différente selon qu'il est adressé à un deuxième professionnel de soins dentaires ou au patient.

De préférence, un message adressé au patient comporte
- une recommandation, par exemple pour prendre un rendez-vous avec un orthodontiste ou pour modifier un traitement orthodontique suivi ; et/ou
- une instruction, par exemple sur la date de la prochaine étape b) à réaliser ; et/ou
- un avis médical sur la configuration de dentition ; et/ou
- des informations sur le traitement orthodontique suivi par le patient, par exemple des photos, de préférence annotées, et/ou des statistiques et/ou des informations permettant une comparaison avec une configuration de dentition antérieure ; et/ou
- des encouragements, par exemple sous la forme de « bons points », accordés en fonction de l'observance du traitement orthodontique et donnant lieu à une récompense ; et/ou
- des statistiques montrant les progrès de l'observance du traitement orthodontique et/ou l'évolution du traitement orthodontique.

De préférence, un message adressé à un deuxième professionnel de soins dentaires comporte une instruction, par exemple pour prendre un rendez-vous avec le patient, pour contacter un autre professionnel de soins dentaires, ou pour modifier un appareil orthodontique destiné au patient.

Dans un mode de réalisation, la valeur actualisée, optionnellement dérivée, définit un statut pour un objectif de traitement. Un procédé selon l'invention est en particulier bien adapté pour communiquer, de manière synthétique sur des objectifs de traitement.

De préférence le statut pour un objectif de traitement est choisi dans un groupe constitué de moins de 10, de préférence moins 5, de préférence moins de 4 statuts potentiels.

Dans un mode de réalisation préféré, le statut d'un objectif de traitement, de préférence pour tout objectif de traitement, est choisi dans un groupe constitué de 3 statuts potentiels. Il précise de préférence si l'objectif de traitement est atteint, n'est pas atteint mais est conforme au plan de traitement, ou n'est pas atteint et n'est pas conforme au plan de traitement. Dans un mode de réalisation, le statut d'un objectif de traitement, de préférence pour tout objectif de traitement, est choisi dans un groupe constitué de 2 statuts potentiels. De préférence, il précise si l'objectif de traitement est atteint ou non à l'instant actualisé.

Avantageusement, un nombre limité de statuts potentiels permet au statut d'être très synthétique.

De préférence, l'élément d'information relatif au statut d'un objectif de traitement présente un format (couleur, taille de caractères, ajout d'un symbole...) différent selon la valeur du statut, par exemple selon que l'objectif de traitement est atteint, n'est pas atteint mais est conforme au plan de traitement, ou n'est pas atteint et n'est pas conforme à un plan de traitement. Cette mise en forme conditionnelle permet avantageusement au professionnel de soins dentaires, à l'étape d), d'identifier immédiatement les objectifs de traitement sur lesquels il doit concentrer son attention, et ainsi, indirectement, accélère le processus d'adaptation éventuelle du traitement orthodontique, et en particulier la réalisation d'un nouvel appareil orthodontique.

De préférence, l'information actualisée déterminée à l'étape c) précise un statut, à l'instant actualisé, pour plus de 1, plus de 2, plus de 5, et/ou moins de 20, moins de 15 objectifs de traitement.

De préférence, pour au moins un, de préférence pour chaque objectif de traitement, l'information actualisée comporte une évaluation du temps restant, à partir de l'instant actualisé, pour atteindre l'objectif de traitement, de préférence conformément à un plan de traitement établi avant l'instant actualisé, de préférence à l'instant de paramétrage, et/ou comporte une indication de la date depuis laquelle l'objectif de traitement est suivi. Le deuxième professionnel de soins dentaires peut ainsi examiner en priorité les objectifs de traitement qui doivent être atteints rapidement. S'il manque de temps, il peut ignorer les autres et reporter leur analyse à un instant actualisé d'une série postérieure d'étapes b) à e).

De préférence, le ou les objectifs de traitement sont choisis dans le groupe suivant :
- le patient atteint une classe d'occlusion n°I pour les canines,
- le patient atteint une classe d'occlusion n°I pour les molaires,
- les espaces du secteur antérieur du patient sont fermés,
- l'espace résultant de l'extraction d'une dent est fermé,
- le patient atteint un surplomb horizontal , ou « overjet » en anglais, normal, de préférence compris entre 1 et 3 mm,
- le patient atteint un surplomb vertical, ou « overbite » en anglais, normal, de préférence compris entre 1 et 3 mm,
- les milieux inter-incisifs des arcades maxillaires et mandibulaires ne sont pas décalés,
- les lignes médianes des arcades inférieure et supérieure ne sont pas décalées,
- le patient ne présente pas de décalage latéral de l'arcade supérieure par rapport à l'arcade inférieure,
- l'arc orthodontique et/ou les appareils auxiliaires sont passifs ;
- pas ou peu de mouvements de dents détectés lors du ou des deux derniers contrôles de l'arcade maxillaire et/ou mandibulaire, de préférence aucun mouvement d'une dent n'a été détecté,
- toutes les dent temporaires sont tombées,
- absence de béance latérale (fermeture de la béance latérale),
- absence de béance postérieure,
- absence de béance antérieure,
- absence d'inversée d'articulé antérieur,
- absence d'inversée d'articulé postérieur,
- amélioration de l'encombrement,
- absence de gingivite,
- absence de tartre,
- amélioration de l'hygiène,
- stabilisation des récession,
- nettoyage de l'appareil orthodontique suffisant,
- diastèmes fermés,
- absence de triangles noirs,
- absence d'infection (par exemple d'aphtes) ou d'irrégularités de la muqueuse,
- amélioration de la teinte/ blancheur des dents.

Le tableau suivant donne des exemples pour le suivi d'objectifs de traitement, les éléments d'information étant sous la forme de feux tricolores :

**[Tableau 1]**

| **Objectif de traitement** | **Attribut** | **Statuts potentiels** | **Eléments d'information** |
|---|---|---|---|
| Classe d'occlusion n°I pour les canines | Occurrence d'une classe d'occlusion n°I pour les canines | Vrai/faux | Feu vert / orange/ rouge ⁽¹⁾ |
| Classe d'occlusion n°I pour les molaires | Occurrence d'une classe d'occlusion n°I pour les molaires | Vrai/faux | Feu vert / orange/ rouge ⁽¹⁾ |
| Espaces antérieurs fermés | Occurrence d'une fermeture des espaces antérieurs | Vrai/faux | Feu vert / orange/ rouge ⁽¹⁾ |
| Tout espace issu d'extraction d'une dent fermé | Occurrence d'une fermeture de tous les espaces issus d'extraction de dent | Vrai/faux | Feu vert / orange/ rouge ⁽¹⁾ |
| Surplomb horizontal normal | Occurrence d'un surplomb horizontal normal | Vrai/faux | Feu vert / orange/ rouge ⁽¹⁾ |
| Surplomb vertical normal | Occurrence d'un surplomb vertical normal | Vrai/faux | Feu vert / orange/ rouge ⁽¹⁾ |
| Lignes médianes des arcades inférieure et supérieure alignées | Alignement des lignes médianes des arcades inférieure et supérieure | Vrai/faux | Feu vert / orange/ rouge ⁽¹⁾ |
| Absence de décalage latéral de l'arcade supérieure par rapport à l'arcade inférieure | Occurrence d'un décalage latéral de l'arcade supérieure par rapport à l'arcade inférieure | Vrai/faux | Feu vert / orange/ rouge ⁽¹⁾ |
| Aucun mouvement d'une dent entre les deux derniers contrôles | Pas d'occurrence d'un mouvement d'une dent entre les deux derniers contrôles | Vrai/faux | Feu vert / orange/ rouge ⁽¹⁾ |
| Absence de dent temporaire | Présence d'une dent temporaire | Vrai/faux | Feu vert / orange/ rouge ⁽¹⁾ |

| | | | |
|---|---|---|---|
| ⁽¹⁾ : vert : objectif atteint ; orange : objectif non atteint et délai à cet effet non dépassé ; rouge : objectif non atteint et délai à cet effet dépassé | | | |

La granulométrie de ces objectifs de traitement est bien adaptée à une analyse rapide de la configuration de dentition du patient.

De préférence, le feu a une couleur différente, par exemple grise, si l'objectif de traitement n'est pas suivi. Dans ce cas, il n'apporte pas d'information sur la configuration de dentition du patient à l'instant actualisé et n'est donc pas un élément d'information exprimant un statut de l'objectif de traitement.

L'étape c) conduit donc à un ensemble d'éléments d'information à afficher, déterminées en fonction de l'analyse de la représentation actualisée et des conditions déterminées à l'étape a).

**A l'étape d),** l'information actualisée déterminée à l'étape c) est communiquée au moins à un deuxième professionnel de soins dentaires, dans les conditions déterminées à l'étape a).

Le deuxième professionnel de soins dentaires peut être identique ou différent du premier professionnel de soins dentaires. En particulier, il peut être un assistant du premier professionnel de soins dentaires.

Chaque objectif de traitement appartient de préférence à un sous-groupe défini en fonction d'un type de mouvement qui fait l'objet de l'objectif de traitement. Le sous-groupe est de préférence choisi parmi les sous-groupes suivants : sous-groupe « antéro-postérieur », sous-groupe « transverse », sous-groupe « vertical » et sous-groupe « autres ». De préférence, à l'étape d), l'information actualisée présentée sur l'écran regroupe les éléments d'information relatifs aux objectifs de traitement dans des zones de l'écran spécifiques aux sous-groupes auxquels ils appartiennent. Avantageusement, ce regroupement facilite la communication de l'information actualisée au deuxième professionnel de soins dentaires à l'étape d).

L'interface de présentation de l'information actualisée est de préférence présentée sur un écran d'un ordinateur de type PC ou sur un écran portable, par exemple sur un écran de téléphone portable.

De préférence, cette interface affiche, au moins un objectif de traitement, de préférence chaque objectif de traitement, un bouton virtuel dont l'activation génère un dialogue pour la modification des conditions de communication, en particulier une plage de communication et/ou une liste de destinataires pour cet objectif de traitement. De préférence, l'interface est également configurée pour une modification d'un critère à utiliser pour l'analyse à l'étape c), par exemple d'une valeur de référence.

De préférence, l'interface affiche également un fil de discussion avec le patient, en particulier entre le patient d'une part et le premier professionnel de soins dentaires et/ou le deuxième professionnel de soins dentaires d'autre part. Avantageusement, le fil de discussion permet au deuxième professionnel de soins dentaires de mieux comprendre les choix réalisés avant l'instant actualisé pour le traitement orthodontique du patient, et donc d'adapter sa décision en conséquence.

De préférence, le fil de discussion est utilisé pour afficher les éléments d'information à destination du patient.

De préférence, l'utilisateur de l'interface peut intervenir dans le fil de discussion, directement depuis l'interface. Avantageusement, il peut ainsi interroger le patient de manière très efficace.

De préférence, au moins une partie de la communication à l'étape d) est réalisée au moyen d'une interface configurée pour la mise en œuvre de l'étape a).

De préférence, à l'étape d), le premier et/ou deuxième professionnel de soins dentaires redéfinissent au moins une partie des conditions de communication pour l'exécution ultérieures d'autres séries d'étapes b) à e).

Dans un mode de réalisation, le traitement orthodontique et/ou un ou plusieurs objectifs de traitement sont modifiés à partir de l'information actualisée communiquée à l'étape d).

**A l'étape e),** postérieure à l'étape d), on réalise ou non, selon l'information actualisée communiquée, une adaptation d'un appareil orthodontique destiné au patient.

Le deuxième professionnel de soins dentaires peut, au regard de l'information actualisée considérer qu'aucune adaptation n'est nécessaire ou qu'une adaptation est nécessaire. En cas d'adaptation, il peut utiliser l'information actualisée et/ou une autre source d'information. En particulier, il peut réaliser des mesures complémentaires sur les dents du patient.

L'adaptation peut consister en la fabrication de tout ou partie d'un nouvel appareil orthodontique, par exemple d'une nouvelle gouttière orthodontique, ou en un changement d'un nouvel arc dentaire, ou en une adaptation d'un appareil orthodontique existant, par exemple d'un appareil orthodontique à arc et attaches.

A l'étape e), un rendez-vous du patient avec le premier et/ou le deuxième professionnel de soins dentaire peut être pris, et/ou l'appareil orthodontique modifié envoyé au patient.

De préférence, les étapes b) à e) sont mises en œuvre de manière cyclique, après une étape a) de préférence exécutée avant le début d'un traitement orthodontique.

De préférence, la série des étapes b) à e) est réalisée plusieurs fois, de préférence à intervalle régulier. L'intervalle temporel entre deux séries est de préférence supérieur à 1 semaine, de préférence supérieur à 2 semaines, et/ou inférieur à 2 mois, de préférence inférieur à 1 mois.

De préférence, au moins une partie des instants actualisés des différentes séries est déterminé avant le début du traitement orthodontique. De préférence, ils correspondent à des instants auxquels une modification ou un changement d'un appareil orthodontique destiné au patient est prévu.

De préférence, avant chaque instant actualisé, on envoie une notification au patient pour lui rappeler la nécessité d'acquérir une représentation actualisée. De préférence, la notification indique une date avant laquelle le patient doit effectuer cette acquisition, cette date étant de préférence écartée de moins de 2 jours de la date d'envoi de la notification.

La notification peut être sous la forme de papier ou, de préférence, sous forme électronique, de préférence adressée au téléphone portable, par exemple sous la forme d'un courriel, d'une alerte automatique d'un applicatif spécialisé mobile ou d'un SMS. Un tel rappel peut être envoyé par le cabinet ou le laboratoire d'orthodontie ou par le premier et/ou deuxième professionnel de soins dentaires ou par un applicatif spécialisé chargé sur le téléphone portable. L'envoi de la notification peut être systématique. Il est de préférence conditionné au fait que le patient n'a pas déjà acquis ladite représentation actualisée.

De préférence, on envoie plusieurs dites notifications successivement, jusqu'à ce que l'utilisateur accuse réception de la notification ou ait acquis la représentation actualisée demandée.

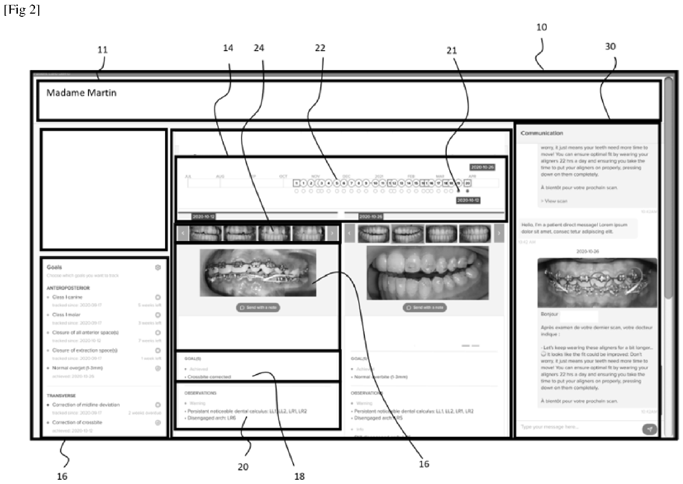

La figure 2 représente une interface 10, notamment destinée aux étapes a) et d).

L'interface présente simultanément sur un écran d'ordinateur :
- un identifiant d'un patient, dans la zone d'identification 11 ; et
- une ligne chronologique 14 représentant des instants successifs sélectionnables individuellement par un opérateur ;
- pour chaque instant sélectionnable sélectionné,
   - au moins une représentation, de préférence extraorale, des arcades dentaires du patient à l'instant sélectionné, ou « représentation principale » 16 et/ou
   - un statut 18, à l'instant sélectionné, pour au moins un objectif de traitement, de préférence le ou les seuls statuts des objectifs de traitement qui, d'après les conditions de communication en vigueur à l'instant sélectionné, devaient être communiqués à cet instant sélectionné et/ou
   - au moins une observation 20 relative à la configuration de dentition du patient à l'instant sélectionné ; et
- de préférence une zone de suivi des objectifs de traitement 32 présentant les éléments d'information relatifs aux statuts des objectifs de traitement de la dernière étape d) réalisée.

La zone d'identification 11 peut en particulier donner le nom et/ou la photo du patient.

La ligne chronologique 14 peut commencer avant l'instant initial marquant le début d'un traitement orthodontique et s'étendre après l'instant final marquant la fin d'un traitement orthodontique. De préférence, elle s'étend depuis l'instant initial marquant le début d'un traitement orthodontique et au moins jusqu'à un instant actualisé d'une étape a) d'un procédé selon l'invention, de préférence sans s'étendre au-delà dudit instant actualisé.

Elle représente une succession d'instants qui peuvent être chacun sélectionnés. La sélection d'un instant, en cliquant sur le rond 21 symbolisant cet instant, permet de visualiser une représentation des arcades dentaires du patient et/ou un élément d'information relatif à un objectif de traitement et/ou une observation relative à la configuration de dentition du patient à cet instant. En sélectionnant un instant sur la ligne chronologique, l'opérateur peut ainsi avantageusement disposer d'une information pertinente pour évaluer la situation dentaire à cet instant.

De préférence, plusieurs instants peuvent être simultanément sélectionnés. Ainsi l'opérateur peut avantageusement comparer des informations pertinentes pour évaluer la situation dentaire aux différents instants sélectionnés. Un instant sélectionné peut être en particulier un instant actualisé d'une étape b). De préférence, l'instant actualisé de la dernière étape b) effectuée est sélectionné par défaut.

La ligne chronologique est de préférence linéaire, c'est-à-dire que la distance entre les représentations de deux instants sur la ligne chronologique, est proportionnelle à l'intervalle temporel entre ces deux instants.

La ligne chronologique représente de préférence, pour chaque instant sélectionnable, un symbole 22 associé relatif à la configuration de dentition du patient audit instant sélectionnable, de préférence relatif au déroulement du traitement orthodontique au regard du plan de traitement. Avantageusement, l'opérateur peut ainsi immédiatement visualiser les périodes pendant lesquelles la situation dentaire présentait une propriété particulière, par exemple visualiser les périodes pendant lesquelles le traitement orthodontique s'est déroulé conformément au plan de traitement et celles où ça n'était pas le cas. L'opérateur peut ainsi très rapidement retrouver des périodes qu'il considère utiles pour son analyse.

Dans un mode de réalisation, la nature de l'information sous-jacente aux symboles 22 de la ligne chronologique peut être modifiée par l'opérateur. Par exemple, l'opérateur peut sélectionner un objectif de traitement de manière que les symboles illustrent la conformité ou la non-conformité du traitement orthodontique au regard du plan de traitement en ne considérant que cet objectif de traitement.

De préférence, tout symbole sur la ligne chronologique est choisi dans un groupe constitué de moins de 10, de préférence moins 5 valeurs, de préférence moins de 4 ou moins de 3 symboles potentiels (c'est-à-dire susceptibles d'être choisis). Dans un mode de réalisation préféré, le symbole est choisi dans un groupe constitué de 2 symboles potentiels indiquant respectivement que le déroulement du traitement orthodontique est conforme et non-conforme au plan de traitement, de manière générale ou pour un objectif de traitement sélectionné.

Pour au moins un, de préférence pour chaque instant sélectionné sur la ligne chronologique, l'interface présente de préférence au moins une observation sur la situation dentaire du patient, la présentation de ladite observation étant de préférence en fonction de l'importance de ladite observation pour le traitement orthodontique suivi par le patient.

La représentation principale 16 est de préférence une image, de préférence une photo, de préférence en couleur réelles, prise, à l'instant sélectionné, par le patient, de préférence avec son téléphone portable, de préférence de manière extra-orale.

De préférence, pour au moins un, de préférence pour chaque instant sélectionné sur la ligne chronologique, l'interface 10 présente une pluralité de miniatures 24 représentant chacune des représentations extraorales de l'arcade dentaire du patient à l'instant sélectionné, de préférence selon des angulations différentes, chaque miniature étant sélectionnable individuellement par l'opérateur, la représentation principale 16 étant une vue agrandie de la miniature sélectionnée.

De préférence, chaque miniature 24 a des dimensions (longueur et largeur) au moins deux fois, de préférence au moins trois fois inférieures à celles de la représentation principale 16. Chaque miniature est de préférence une image, de préférence une photo, de préférence en couleur réelles, prise, à l'instant sélectionné, par le patient, de préférence avec son téléphone portable, de préférence de manière extra-orale, de préférence lors d'une même étape b).

De préférence, tous les instants simultanément sélectionnés sur la ligne chronologique sont traités de manière identique, en particulier pour afficher à chaque fois, pour l'instant considéré, une représentation principale 16 et de préférence des miniatures 24, et/ou un élément d'information pour au moins un objectif de traitement, et/ou au moins une observation sur la configuration de dentition du patient.

De préférence encore, l'interface 10 affiche un fil de discussion 30 avec le patient, en particulier entre le patient et l'opérateur.

De préférence toujours, l'interface 10 affiche des informations générales sur le traitement, en particulier une date de début du traitement et/ou la date de la prochaine acquisition d'images actualisées par le patient.

De préférence, toute sélection d'un objet représenté sur l'interface (symboles et/ou miniatures notamment) est possible en cliquant sur l'objet.

Une interface 10 selon l'invention peut avantageusement être mise en œuvre à l'étape d) de communication de l'information actualisée, l'opérateur étant le deuxième professionnel de soins dentaires.

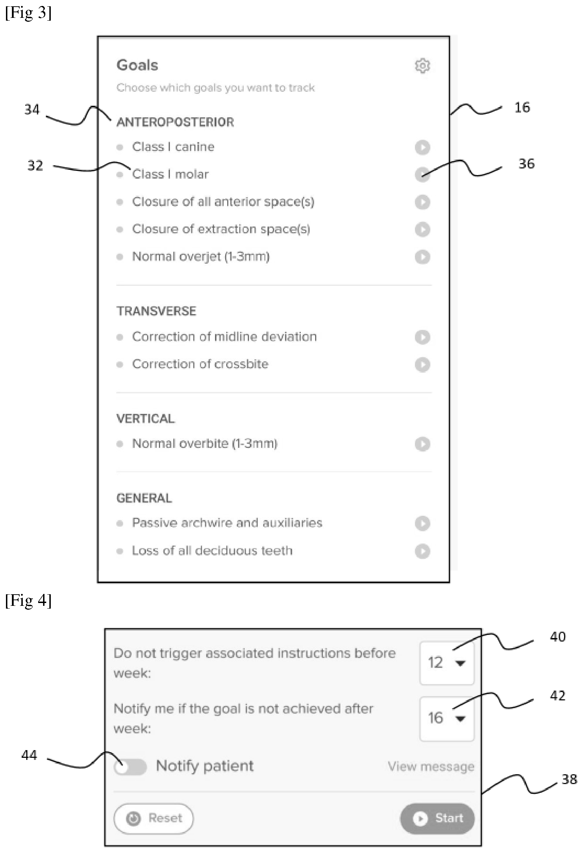

Elle comporte de préférence une zone de suivi des objectifs 32, représentée plus en détail sur la figure 3, qui présente les éléments d'information pour les différents objectifs de traitement déterminés lors de la dernière exécution d'une étape c).

La zone de suivi des objectifs 32 liste tous les objectifs de traitement 34, regroupés dans des dits sous-groupes 34.

A chaque objectif de traitement est associée une marque sous la forme d'un point, dont la couleur indique le statut de l'objectif de traitement, de préférence suivant la nomenclature suivante :
- gris : l'objectif de traitement n'est pas suivi ;
- bleu : l'objectif de traitement est suivi, n'est pas encore atteint, et la limite pour atteindre cet objectif de traitement n'est pas atteinte ;
- vert : l'objectif de traitement est atteint ;
- rouge : l'objectif de traitement est suivi, n'est pas encore atteint, et la limite pour atteindre cet objectif de traitement est dépassée.

A chaque objectif de traitement est associé un bouton virtuel 36. L'appui sur ce bouton provoque l'ouverture de la boîte de dialogue représentée 38 sur la figure 4. L'opérateur peut alors préciser des dates de début 40 et de fin 42 pour une plage de communication et si le patient doit être informé du statut de l'objectif, en déplaçant le curseur 44.

### Système

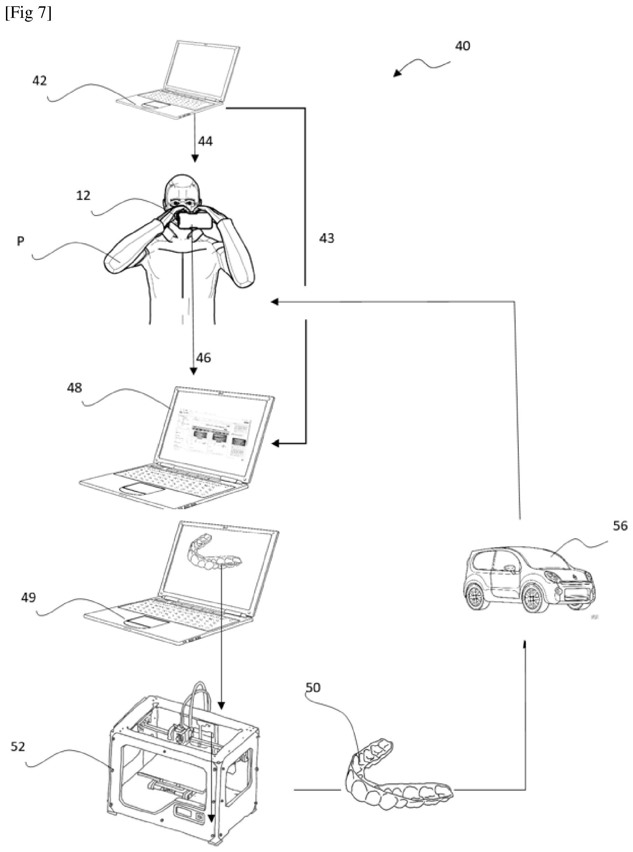

La figure 7 illustre un exemple d'un système selon l'invention et illustre la mise en œuvre d'un procédé selon l'invention :
Ce système 40 comporte :
- de préférence un premier ordinateur 42, paramétré par le premier professionnel de soins dentaires pour définir les conditions de communication 43, et de préférence configuré pour envoyer au patient P un rappel 44 pour qu'il acquiert une représentation actualisée 46 avec un téléphone portable 45, en l'occurrence fixé sur un embout buccal du type de celui représenté sur la figure 8 ;
- le téléphone portable 45, configuré pour acquérir ladite représentation actualisée 46 ;
- un deuxième ordinateur 48, identique ou différent du premier ordinateur 42, de préférence distant et en communication avec le téléphone portable 45,
   apte à recevoir et à analyser ladite représentation actualisée 46 du téléphone portable pour déterminer une information actualisée,
   apte à recevoir les conditions de communication 43 du premier ordinateur 42, et apte à présenter l'information actualisée à un deuxième professionnel de soins dentaires selon les conditions de communication 43 ;
- un troisième ordinateur 49 apte à concevoir ou à modifier, de préférence avec l'aide du deuxième professionnel de soins dentaires, un appareil orthodontique, par exemple une gouttière orthodontique 50 ;
- optionnellement, une machine 52 de fabrication de l'appareil orthodontique, par exemple apte à fabriquer la gouttière orthodontique 50 conçue avec le troisième ordinateur, de préférence à partir d'un modèle généré par ledit troisième ordinateur ;
- de préférence un réseau de transport 56 apte à transporter l'appareil orthodontique, par exemple la gouttière orthodontique 50, depuis ladite machine de fabrication jusqu'au patient P.

Comme cela apparaît clairement à présent, l'invention fournit une solution pour améliorer l'efficacité de la communication à destination du ou des deuxièmes professionnels de soins dentaires. L'analyse de la situation dentaire en est améliorée, et donc l'adaptation de l'appareil orthodontique est plus précise.

L'invention peut être en particulier utilisée au moins pour :
- suivre un mouvement de la mâchoire inférieure par rapport à la mâchoire supérieure, et/ou
- détecter une position anormale de la mâchoire inférieure par rapport à la mâchoire supérieure, et/ou
- suivre une évolution de la forme de la mâchoire inférieure et/ou de la mâchoire supérieure, et/ou
- détecter une forme anormale de la forme de la mâchoire inférieure et/ou de la mâchoire supérieure.

Bien entendu, l'invention n'est pas limitée aux modes de réalisation décrits en détail ci-dessus.

## Revendications

1. Système pour la mise en œuvre d'un procédé d'adaptation d'un appareil orthodontique destiné à un patient, ledit procédé comportant les étapes suivantes :
a) à un instant de paramétrage, paramétrage, par un premier professionnel de soins dentaires et spécifiquement pour ledit patient, de conditions pour une communication, par ordinateur, d'une information actualisée relative à la configuration de dentition du patient ;
c) analyse d'une représentation actualisée, de manière à déterminer une information sur ladite configuration de dentition à l'instant actualisé, ou « information actualisée »,
ladite représentation actualisée étant une représentation extraorale d'au moins une partie des arcades dentaires du patient acquise à un instant actualisé postérieur à l'instant de paramétrage, au moyen d'un appareil d'acquisition, par le patient ou un proche du patient, et étant constituée d'un ensemble d'images et/ou d'un modèle numérique tridimensionnel,
ladite information actualisée étant constituée d'un ensemble d'éléments d'information exprimant chacun un statut pour un objectif de traitement respectif, au regard de la représentation actualisée, un statut ne pouvant prendre qu'un nombre limité de valeurs, dites « statuts potentiels », un statut à l'instant actualisé, égal à un des statuts potentiels pour l'objectif de traitement, constituant une « valeur actualisée » ;
d) communication de l'information actualisée à au moins un deuxième professionnel de soins dentaires, dans les conditions déterminées à l'étape a), lesdites conditions définissant, pour chaque élément d'information si ledit élément d'information doit être communiqué à l'étape d) ;
e) optionnellement, en fonction de l'information actualisée communiquée à l'étape d), adaptation ou non dudit appareil orthodontique,
ledit système comportant :
- un premier ordinateur (42), paramétré, à l'étape a), par le premier professionnel de soins dentaires pour définir les conditions de communication (43) de l'information actualisée ;
- un deuxième ordinateur (48), identique ou différent du premier ordinateur (42) apte à recevoir et à analyser ladite représentation actualisée (46) de l'appareil d'acquisition pour déterminer une information actualisée à l'étape c),
apte à recevoir les conditions de communication (43) du premier ordinateur (42), et apte à présenter l'information actualisée au deuxième professionnel de soins dentaires selon les conditions de communication (43), à l'étape d),
les conditions déterminées à l'étape a) définissant, pour chaque élément d'information si ledit élément d'information doit être communiqué à l'étape d), en fonction de l'instant actualisé et/ou de la valeur actualisée associée à l'élément d'information et/ou de l'âge du patient et/ou du traitement orthodontique ou du type de traitement orthodontique suivi par le patient, et/ou du statut d'un ou plusieurs objectifs de traitement.

2. Système selon la revendication immédiatement précédente, dans lequel le deuxième professionnel de soins dentaires est identique au premier professionnel de soins dentaires.

3. Système selon l'une quelconque des revendications précédentes, dans lequel les conditions déterminées à l'étape a) fixent pour au moins un dit objectif de traitement,
- au moins une plage de communication, exclusivement pendant laquelle l'élément d'information correspondant, déterminé à l'étape c), est communicable à l'étape d) ; et/ou
- une fréquence maximale de communication de l'élément d'information.

4. Système selon la revendication immédiatement précédente, dans lequel les conditions déterminées à l'étape a) fixent pour ledit objectif de traitement, des dites plages de communication différentes en fonction du statut dudit objectif de traitement.

5. Système selon l'une quelconque des revendications précédentes, dans lequel les conditions de communication d'au moins un élément d'information déterminées à l'étape a) sont en fonction de l'objectif de traitement concerné et/ou du statut de l'objectif de traitement exprimé par ledit élément d'information et/ou du destinataire de l'élément d'information.

6. Système selon l'une quelconque des revendications précédentes, dans lequel le statut d'un objectif de traitement, de préférence d'un objectif de traitement quelconque est choisi dans un groupe de statuts potentiels comportant moins de 10 statuts potentiels.

7. Système selon l'une quelconque des revendications précédentes, dans lequel le nombre d'objectifs de traitement est inférieur à 30.

8. Système selon l'une quelconque des revendications précédentes, dans lequel pour au moins un, de préférence pour chaque objectif de traitement, l'information actualisée comporte une évaluation du temps restant, à partir de l'instant actualisé, pour atteindre l'objectif de traitement conformément à un plan de traitement établi avant l'instant actualisé, et/ou comporte une indication de la date depuis laquelle l'objectif de traitement est suivi.

9. Système selon l'une quelconque des revendications précédentes, dans lequel le deuxième ordinateur comporte un réseau de neurones convolutif configuré pour analyser ladite représentation actualisée (46) pour déterminer une information actualisée à l'étape c).

10. Système selon l'une quelconque des revendications précédentes, dans lequel les premier et deuxième ordinateur présentent une même interface d'ordinateur pour le paramétrage à l'étape a) et la communication à l'étape d).

11. Système selon la revendication immédiatement précédente, dans lequel l'interface présente simultanément :
- un identifiant du patient ; et
- une ligne chronologique représentant des instants successifs sélectionnables individuellement par un opérateur ; et
- pour chaque instant sélectionnable sélectionné, au moins une représentation extraorale des arcades dentaires du patient à l'instant sélectionné, ou « représentation principale » et/ou un élément d'information exprimant un statut, à l'instant sélectionné, pour au moins un objectif de traitement et/ou au moins une observation relative à la configuration de dentition du patient à l'instant sélectionné ; et
- une zone de suivi des objectifs de traitement fournissant des éléments d'information exprimant des statuts desdits objectifs de traitement à l'instant où l'interface est affichée.

12. Système selon l'une quelconque des revendications précédentes, comportant
- un troisième ordinateur (49), identique ou différent des premier et deuxième ordinateur, configuré pour, en fonction de l'information actualisée communiquée à l'étape d), déterminer l'adaptation de l'appareil orthodontique à l'étape d), automatiquement ou, de préférence, avec l'assistance du deuxième professionnel de soins dentaires ; et/ou
- une machine (52) de fabrication dudit appareil orthodontique ;
- optionnellement, un réseau de transport (56) apte à transporter ledit appareil orthodontique (50) depuis ladite machine de fabrication jusqu'au patient (P).

13. Système selon l'une quelconque des revendications précédentes, dans lequel ledit objectif de traitement est choisi dans le groupe suivant :
- appartenance de l'occlusion à une classe d'occlusion prédéfinie, chaque classe d'occlusion définissant une relation inter-arcades sur le plan sagittal,
- espacement des dents du secteur antérieur du patient inférieurs à des espaces prédéfinis, de préférence nuls,
- espace résultant de l'extraction d'une dent du patient inférieur à un espace prédéfini, de préférence nul,
- surplomb horizontal inférieur à un surplomb horizontal prédéfini,
- surplomb vertical inférieur à un surplomb vertical prédéfini,
- décalage des milieux inter-incisifs des arcades maxillaires et mandibulaires inférieur à un décalage prédéfini, de préférence nul,
- décalage des lignes médianes des arcades inférieure et supérieure du patient inférieur à un décalage prédéfini, de préférence nul,
- décalage latéral de l'arcade supérieure par rapport à l'arcade inférieure inférieur à un décalage prédéfini, de préférence nul,
- niveau d'activité de l'appareil orthodontique inférieur à un niveau prédéfini, de préférence nul ;
- vitesse d'évolution des mouvements des dents inférieure à un niveau prédéfini ;
- amplitude d'un mouvement d'une ou plusieurs dents de l'arcade maxillaire et/ou mandibulaire du patient, depuis un contrôle antérieur, inférieure à une amplitude prédéfinie,
- correction de la rotation d'une ou plusieurs dents atteinte,
- alignement des dents d'une arcade sur le plan occlusal atteint,
- nivellement des dents d'une arcade atteint,
- niveau d'abrasion d'une ou plusieurs dents inférieur à un niveau prédéfini,
- nombre de dents temporaires du patient tombées inférieur à un nombre prédéfini,
- niveau de béance latérale inférieur à un niveau prédéfini, de préférence nul,
- niveau de béance postérieure inférieur à un niveau prédéfini, de préférence nul,
- niveau de béance antérieure inférieur à un niveau prédéfini, de préférence nul,
- absence d'inversée d'articulé antérieur,
- absence d'inversée d'articulé postérieur,
- encombrement inférieur à un encombrement prédéfini,
- absence de gingivite,
- absence de péri-implantite,
- taux de tartre inférieur à un taux prédéfini,
- mesure du niveau d'hygiène supérieure à une mesure du niveau d'hygiène prédéfinie,
- valeur de récession dans une plage prédéfinie,
- fermeture d'espace ou correction de diastème inférieur à un niveau prédéfini, de préférence nul,
- niveau de diastème inférieur à un niveau prédéfini, de préférence nul,
- absence de triangles noirs,
- absence d'infection, en particulier absence d'aphtes, ou d'irrégularités de la muqueuse,
- valeur de la teinte/ blancheur des dents dans une plage prédéfinie.

14. Ordinateur comportant un support informatique dans lequel est enregistré un programme d'ordinateur comprenant des instructions de code de programme pour mettre en œuvre les étapes suivantes :
a) à un instant de paramétrage, détermination, par un premier professionnel de soins dentaires et spécifiquement pour ledit patient, de conditions pour une communication, par ordinateur, d'une information actualisée relative à la configuration de dentition du patient puis saisie desdites conditions de communication dans un ordinateur ;
c) analyse d'une représentation actualisée, de manière à déterminer une information sur ladite configuration de dentition à l'instant actualisé, ou « information actualisée »,
ladite représentation actualisée étant une représentation extraorale d'au moins une partie des arcades dentaires du patient acquise à un instant actualisé postérieur à l'instant de paramétrage, au moyen d'un appareil d'acquisition, par le patient ou un proche du patient, et étant constituée d'un ensemble d'images et/ou d'un modèle numérique tridimensionnel,
ladite information actualisée étant constituée d'un ensemble d'éléments d'information exprimant chacun un statut pour un objectif de traitement respectif, au regard de la représentation actualisée, un statut ne pouvant prendre qu'un nombre limité de valeurs, dites « statuts potentiels », un statut à l'instant actualisé, égal à un des statuts potentiels pour l'objectif de traitement, constituant une « valeur actualisée » ;
d) communication de l'information actualisée à au moins un deuxième professionnel de soins dentaires, dans les conditions déterminées à l'étape a), lesdites conditions définissant, pour chaque élément d'information si ledit élément d'information doit être communiqué à l'étape d) ;
